# EUROPEAN PATENT SPECIFICATION

(11) **EP 3 520 716 B1**
(45) Date of publication and mention of the grant of the patent: **02.09.2020**
(21) Application number: 17854538.0
(22) Date of filing: 17.07.2017
(51) Int. Cl.: A61B 17/11, A61B 17/115, A61B 17/00

(54) **ABSORBABLE AND UNIDIRECTIONALLY COMPRESSIBLE INTESTINE-INTESTINE STAPLER**
RESORBIERBARES UND UNIDIREKTIONAL KOMPRIMIERBARES DARM-DARM-KLAMMERGERÄT
AGRAFEUSE POUR ANASTOMOSE D'INTESTIN RÉSORBABLE ET COMPRESSIBLE DE MANIÈRE UNIDIRECTIONNELLE

(30) Priority: 29.09.2016 CN 201610863689
(43) Date of publication of application: 07.08.2019
(73) Proprietor: Zhejiang University, Hangzhou, Zhejiang 310027 (CN)
(72) Inventor: CAI, Xiujun, Hangzhou Zhejiang 310012 (CN); CHEN, Mingyu, Hangzhou Zhejiang 310012 (CN); LU, Chen, Hangzhou Zhejiang 310012 (CN); WANG, Yifan, Hangzhou Zhejiang 310012 (CN); HUANG, Diyu, Hangzhou Zhejiang 310012 (CN); ZHU, Hepan, Hangzhou Zhejiang 310012 (CN); ZHU, Yibin, Hangzhou Zhejiang 310012 (CN); ZHANG, Bin, Hangzhou Zhejiang 310012 (CN)
(74) Representative: Rössler, Matthias
(86) International application number: PCT/CN2017/093163
(87) International publication number: WO 2018/059084

(56) References cited:
- EP-A2- 1 797 831
- WO-A1-02/096299
- CN-A- 102 112 069
- CN-A- 105 078 535
- CN-A- 105 120 773
- CN-A- 106 308 866
- CN-U- 203 970 452
- CN-U- 203 970 467
- CN-U- 206 403 818
- US-A- 4 055 186
- US-A- 4 552 148
- US-A- 5 336 233
- US-A1- 2013 110 140

## Description

The present invention relates to an absorbable and unidirectionally compressive intestinal stapler.

Intestinal anastomosis is one of the most common operations in abdominal surgery, and it is also the surgical technique that surgeons must master. It is the key technology for digestive tract reconstruction and is related to the success or failure of surgery. Ideal bowel anastomosis should avoid complications such as anastomotic leakage, stenosis, hemorrhage, etc., and is easy to operate. Improvement and innovation matching technology has always been a hot topic in external scientific research. Up to now, hundreds of anastomotic methods have been developed. These methods have their own advantages and disadvantages, but none of them can fully meet the above requirements. For example, the most commonly used manual suture method and stapler method in clinical practice cannot completely avoid complications such as anastomotic leakage, stenosis and hemorrhage.
In US 2013 /110140 A1 an anastomosis system is disclosed including a base, two rings, and a blood vessel-leading unit. A first blood vessel can be inserted in and attached to the first ring by the blood vessel-leading unit and a second blood vessel can be inserted in and attached to the second ring by the blood vessel-leading unit. The first ring is placed on a ring-guiding base in a specific manner. The second ring is placed on the ring-guiding base in a movable and rotatable manner to assure the alignment of the blood vessels in both axial and angular directions. The two rings are interconnected by at least one snap-on mechanism or buckle so that the interconnection of the rings is efficient.

The US 5 336 233 A describes an anastomotic device, especially adapted for anastomosis by intraluminal disposition and without the intervention of additional mechanical holding or clamping means, comprising: a pair of annuli; each annulus of the pair being a relatively thin, flat ring having a plurality of impaling pins integral therewith and extending circumferentially and normally from one face thereof. Thereby each pin being pointed at its end to facilitate an impaling function and being transversely serrated at an end section remote from the ring. Each said rings having a plurality of apertures therethrough and said pins and apertures circumferentially alternating with one another. Said one face of each annulus being beveled about each said aperture for a pin guiding function and each aperture having therein a relatively thin flexible tang that is an extension from the inner periphery of the aperture defining annulus structure. Further an alignment indicator element integral with and extending from a periphery of each said ring is provided for. The parts being configured and dimensioned whereby with the parts to be anastomosed each mounted on and about a respective ring member and the indicator elements aligned. The rings are operatively associated with one another for anastomosis whereby each pin is guided and received within an opposed operatively associated aperture, with the tang of said associated aperture lockingly engaged with a said serration. Said anastomosed parts are between operatively associated flat surfaces of said annuli.

As disclosed in US 4 055 186 A an anastomosis button is formed of a pair of axially engaged complementary clamping members, each clamping member includes a hub and a crown. The crown includes a clamping collar which is coaxially positioned about and spaced outwardly of the hub medially along the length of the hub and a plurality of spring leaves which are spaced apart and attached at one set of ends to the hub and at the other set of ends to the clamping collar.

An anastomotic device surgically joins the free ends of a first and a second tubular structure is discussed in EP 1 797 831 A2. The device generally includes two ring members, each of which is secured to a free end of the tubular structure. Each ring member includes an outer portion and an inner portion. The outer portions are each cylindrical and ring-like with open ends. The length of the cylindrical outer portions provides support for the portions of the tubular structures near and adjacent the ends of the tubular structure, thus reducing the likelihood of stricture during healing of the anastomotic tissue. Outer portions have holes around their perimeters which provide a scaffold for tissue to grow through for increasing the strength of the tissue near the anastomosis. The inner portions have connecting means and are designed to matingly engage one another.

Another anastomotic device is disclosed in WO02096299 A1. This anastomotic device adopted to surgically join the free ends of a first and a second tubular structure. The device generally includes two ring members, each of which is secured to a free end of the tubular structure. Each ring member includes an outer portion and an inner portion. The outer portions are each cylindrical and ring-like with open ends. The length of the cylindrical outer portions provides support for the portions of the tubular structures near and adjacent the ends of the tubular structure, thus reducing the likelihood of stricture during healing of the anastomotic tissue. Outer portions have holes around their perimeters which provide a scaffold for tissue to grow through for increasing the strength of the tissue near the anastomosis. The inner portions have connecting means and are designed to matingly engage one another.

In CN 203 970 467 U a biodegradable intestine anastomosis ring is disclosed, which comprises two identical anastomosis pieces made from macromolecular biodegradable polylactic acid, wherein each anastomosis piece comprises a tubular bracket body and at least two strip-shaped locking rods. One end of each strip-shaped locking rod is connected with the bracket body and the locking rods are uniformly distributed along the internal circumference of the bracket body. Inverted-hooked beveled elastic locking keys are arranged at the top ends of the locking rods and the inner wall and the outer wall of the bracket body are smooth. Notches matched with the elastic locking keys arranged at the top ends of the locking rods are arranged at the tail end of the bracket body. The biodegradable intestine anastomosis ring can remarkably increase intestine anastomosis speed, effectively protects an anastomotic stoma from being polluted by an intestinal content, and reduces anastomosis postoperative complications.

Other techniques involve devices like those disclosed in CN 105 078 535 A and CN 105 120 773 A.

At present, the traditional intestinal suture anastomosis and tubular stapler are mainly used.

Conventional manual suture anastomosis, that is, the surgeon uses a conventional surgical method to manually suture and knot with a needle holder, forceps, and needle thread.

The tube-type stapler, that is, the tube-type stapler, is placed in one end of the intestine tube, and the other end of the excitation rod is placed again, and the end-end anastomosis is performed, but the tube-type stapler is mainly applied to the rectum and part of the sigmoid colon. Intestinal anastomosis requires an additional small opening and suturing the small opening.

The linear cutting and closing device improves the intestinal anastomosis. This method can correct the end-to-end anastomosis of the intestine, but it changes the original structure of the digestive tract.

In summary, the traditional intestinal anastomosis has the disadvantages of high operational difficulty and high incidence of postoperative anastomotic leakage; while the tubular stapler is suitable for a small range of intestinal tubes; in addition, the above traditional anastomosis is more difficult to be performed under laparoscopy, and the main problem of anastomotic leakage can't be effectively solved.

In order to overcome the above-mentioned defects of the existing anastomosis manner, the present invention provides an absorbable and unidirectionally compressive intestinal stapler for reducing the incidence of anastomotic leakage, reducing the difficulty of intestinal anastomosis, accelerating the progress of the operation, especially the difficulty of laparoscopic intestinal anastomosis, and shortening the operation time, Expandable intestinal anastomosis, especially expanding the scope of application of the absorbable and unidirectional compressive end-to-end anastomosis.

The technical solution adopted by the invention is:
an absorbable and unidirectionally compressive intestinal stapler as defined by the features of the independent claim. Preferred embodiments are given in the dependent claims.

The beneficial effects of the present invention are:
1. Two anastomosed intestines in the embodiment are relatively static, so the trouble that the intestinal canal needs to be adjusted when being matched with other assembling modes is avoided;
2. The operation is simple, the success rate is high, the wound is small, and the original physiological result of the digestive tract is ensured as much as possible, and the intestines are anastomosed with the end to end, while has the characteristic of obviously reducing the incidence rate of the anastomotic leakage after intestinal anastomosis.
3. The invention can be applied for both open surgery and laparoscopic surgery.
4. The invention can be applied for a wider range of bowel, such as the small intestine, colon, sigmoid colon and rectum.
5. The invention can significantly shorten the time of intestinal anastomosis, and can reduce the difficulty of laparoscopic suture, and it is suitable for beginners.

Fig. 1 is a view of an integral structure of the present invention.
Fig.2 and Fig.3 are perspective views of the overall structure of the present invention.
Fig.4 is a view showing a state in which the first through pipe portion of the present invention is unfolded with respect to the second through pipe portion.
Fig. 5 is a schematic plan view showing the state in which the first through-pipe portion of Fig. 4 is unfolded with respect to the second through-tube portion.
Fig.6 and Fig.7 are schematic perspective views of the annular structure of the present invention, respectively.
Fig.8 is a schematic view showing the planar structure of the annular structure of the present invention.

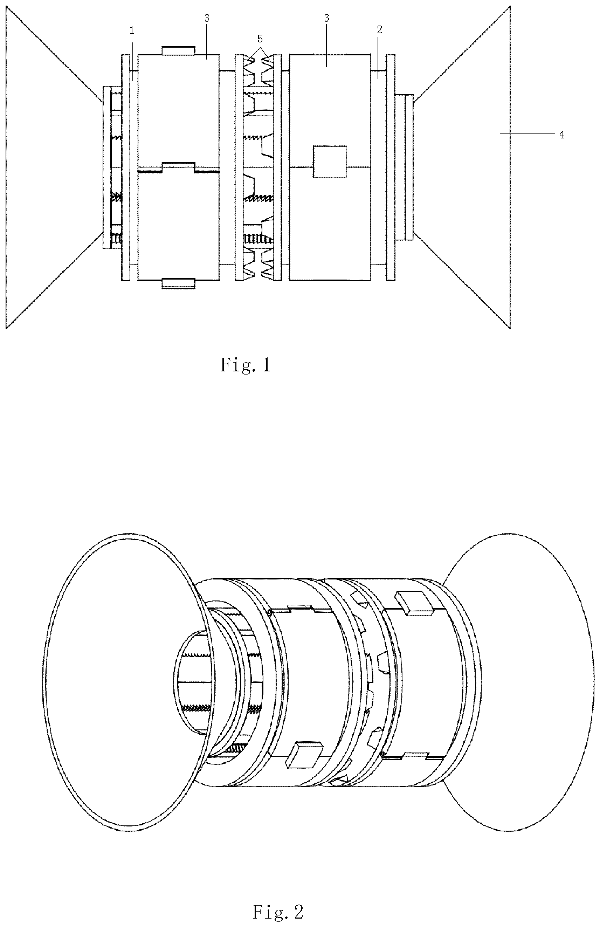

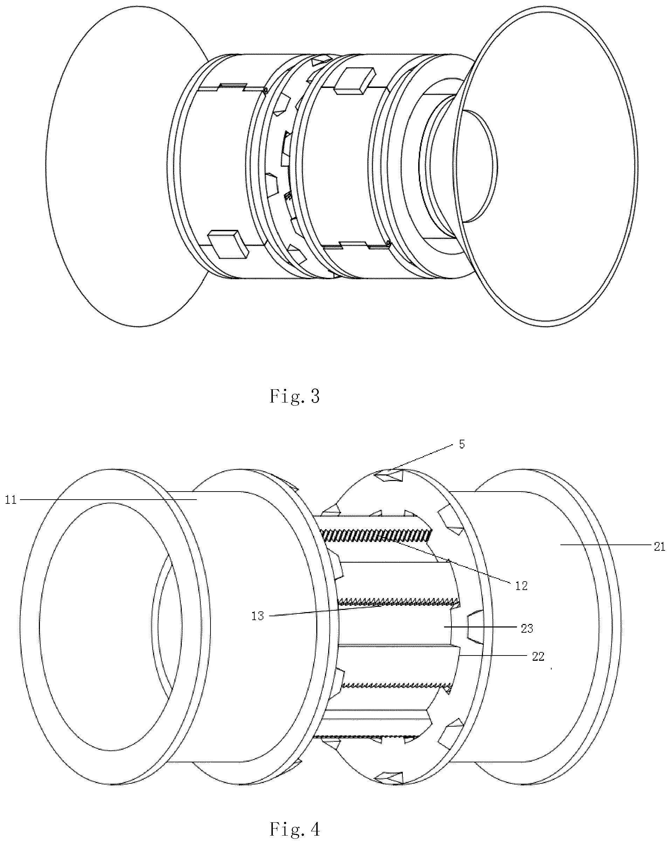

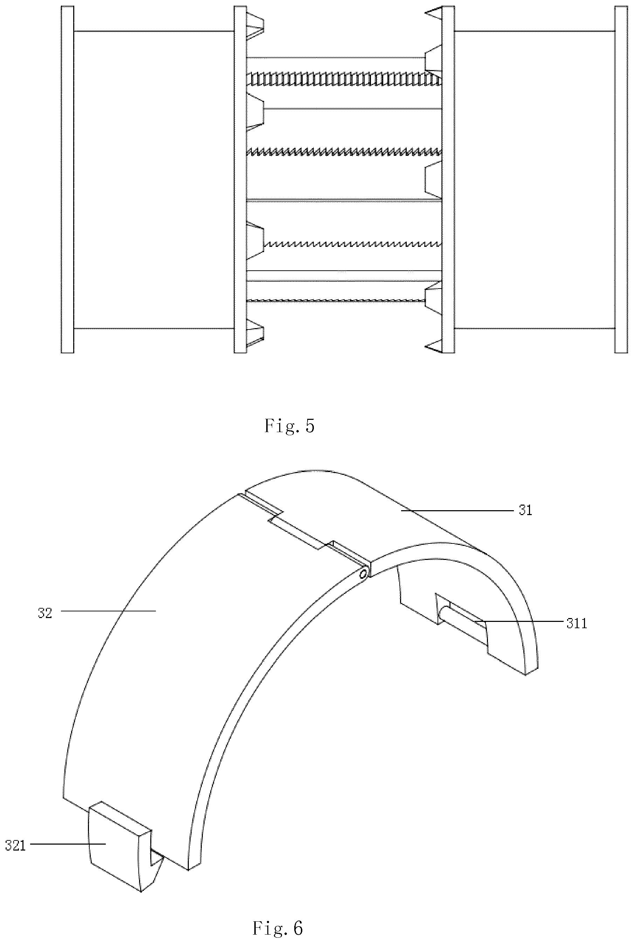

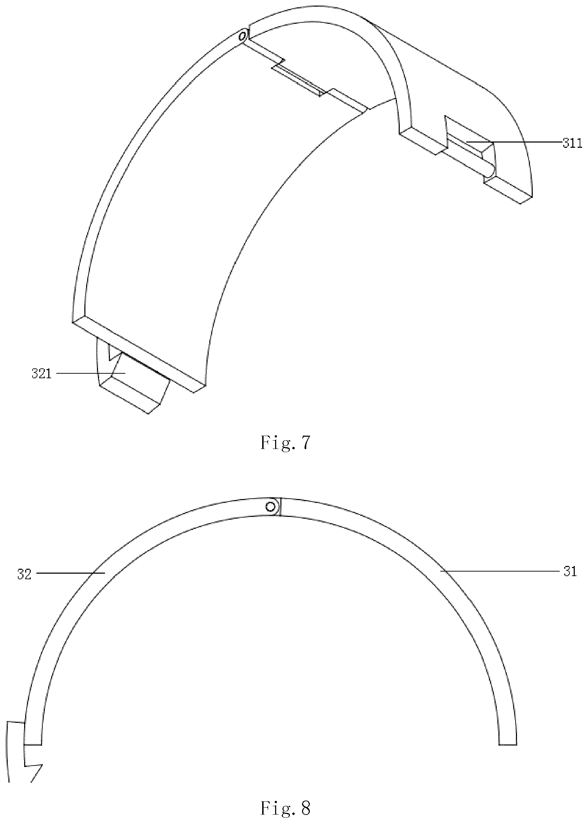

With Reference to Fig.1 to Fig.8, an absorbable and unidirectionally compressive intestinal stapler is described, comprising a first through pipe portion 1 and a second through pipe portion 2 which are matched mutually and which are made of a human acceptable degradation material; the first through pipe portion 1 can move back and forth relative to the second pipe portion 2 along a common axial direction thereof, and the first through pipe 1 and the second through pipe 2 are inseparable; The through pipe portion 1 and the second through pipe portion 2 respectively comprise a large end (11,21) and a small end, and the large ends (11,21) is for fixing the intestinal canal, and the small end of the second through pipe portion 2 is sheathed into the small end of the first through pipe portion 1. The two large ends (11, 21) are consistent in size; when the first through pipe portion (1) moves relative to the second through pipe portion (2), the two large ends (11, 21) are draw close to each other to achieve an intestine-intestine and end-end anastomosis.

Each of the inner side of the large end of the first through pipe portion 1 and the inner side of the large end of the second through pipe portion 2 is provided a circle of protrusion, and a plurality of through grooves 22 are provided on the inner side of the wall of the large end of the second through pipe portion 2 at intervals in the circumferential direction, a plurality of first racks 12 at intervals in the circumferential direction form the wall of the small end of the first through pipe portion 1 in the axial direction, the first rack 12 penetrates through the through groove 22 and is movable along the through groove 22;

A plurality of second racks 23 at intervals in the circumferential direction form the wall of the small end of the second through pipe portion 2, and there is a sliding groove 13 at every abutment of the first racks 12 and the second racks 23; the second rack 23 and the first rack 12 are arranged in a staggered mode, the first rack 12 and the second rack 23 are matched to form a closed space;

The tail ends of the first racks 12 and the second racks 23 are connected through a circular ring respectively;

The outer side of circular ring at the ends of the small end of the first through pipe portion (1) and that of the second through pipe portion (2) are provided with gradually-expanded elastic flared openings (4) respectively; when the first through pipe portion (1) closes up or opens relative to the second through pipe portion (2), the elastic flared openings (4) of the two ends open or are squeezed into the large ends (11,) of the first through pipe portion (1) or the large ends (21) of the second through pipe portion (2) respectively.

The invention further comprises an annular structure 3 which is arranged in the circumferential direction and is used for reinforcing the anastomotic site, the annular structure 3 comprises a first edge 31 and a second edge 32, and one end of the first edge 31 is hinged to one end of the second edge 32, the other end of the first edge 31 is connected with the other end of the second edge 32 in a clamped mode.

A clamping groove 311 is provided in the other end of the first edge 31, and the other end of the second edge 32 is provided with a buckle 321, the other end of the first edge 31 and the other end of the second edge 32 are connected with the buckle 321 fastening a clamping groove 311 in a matched mode.

The inner side of the large end is provided with a plurality of tooth-shaped structures 5 for preventing the intestinal canal from slipping off.

The elastic flared opening 4 is detachable.

The operation process of the aspect of the disclosure is as follows:
①Pre-measuring the diameter of an intestinal canal to be matched;
②Two sides intestinal canal are respectively arranged at two large ends of the anastomat, and the edge of the intestinal canal is buckled on the tooth-shaped structure of the anastomotic position;
③The two sections of intestines are respectively fastened with an annular structure for reinforcing;
④Fourthly, the two sections of intestinal pipes are closed up to the middle, so that the intestinal tract anastomosis is directly completed, and the alignment is accurate.

Two anastomosed intestines in the embodiment are relatively static, so the trouble that the intestinal canal needs to be adjusted when being matched with other assembling modes is avoided, and the alignment is accurate. Moreover, the operation is simple, the success rate is high, the wound is small, and the original physiological result of the digestive tract is ensured as much as possible, and the intestines are anastomosed with the end to end, while has the characteristic of obviously reducing the incidence rate of the anastomotic leakage after intestinal anastomosis.

The contents of the embodiments of the present invention are just a list of implementation forms of the inventive concept, the scope of the invention should not be considered as limited to the specific forms set forth in the embodiments.

## Claims

1. An absorbable and unidirectionally compressive intestinal stapler, comprising a first through pipe portion (1) and a second through pipe portion (2) which are matched mutually and which are made of a human acceptable degradation material; wherein, the first through pipe portion (1) can move back and forth relative to the second through pipe portion (2) along a common axial direction thereof; the first through pipe portion(1) and the second through pipe portion (2) are inseparable; the first through pipe portion (1) and the second through pipe portion (2), respectively, comprise a large end (11,21) and a small end, and the large ends (11,21) are for fixing the intestinal canal, and the small end of the second through pipe portion (2) is sheathed into the small end of the first through pipe portion (1); the two large ends (11, 21) are consistent in size; when the first through pipe portion (1) moves relative to the second through pipe portion (2), the two large ends (11, 21) are drawn close to each other to achieve an intestine-intestine and end-to-end anastomosis; on each of an inner side of the large end of the first through pipe portion (1) and an inner side of the large end of the second through pipe portion (2) is provided a circle of protrusions, and a plurality of through grooves (22) are provided on an inner side of a wall of the large end of the second through pipe portion (2) at intervals in the circumferential direction, a plurality of first racks (12) at intervals in the circumferential direction form a wall of the small end of the first through pipe portion (1) in the axial direction, the first rack (12) penetrates through the through groove (22) and is movable along the through groove (22);
a plurality of second racks (23) at intervals in the circumferential direction form a wall of the small end of the second through pipe portion (2), and there is a sliding groove (13) at every abutment of the first racks (12) and the second racks (23); the second racks (23) and the first racks (12) are arranged in a staggered mode, the first racks (12) and the second racks (23) are matched to form a closed space;
the tail ends of the first racks (12) and the second racks (23) are connected through a circular ring, respectively; the outer sides of the circular rings at the ends of the small ends of the first through pipe portion (1) and that of the second through pipe portion (2) are provided with gradually expandable elastic flared openings (4), respectively; when the first through pipe portion (1) closes up or opens relative to the second through pipe portion (2), the elastic flared openings (4) of the two ends open or are squeezed into the large end (11) of the first through pipe portion (1) and the large end (21) of the second through pipe portion (2) respectively.

2. An absorbable and unidirectionally compressive intestinal stapler according to claim 1, further comprising an annular structure (3) which is arranged in the circumferential direction and is used for reinforcing the anastomotic site, the annular structure (3) comprises a first edge (31) and a second edge (32), and one end of the first edge (31) is hinged to one end of the second edge (32), the other end of the first edge (31) is connected with the other end of the second edge (32) in a clamped mode.

3. An absorbable and unidirectionally compressive intestinal stapler according to claim 2, wherein a clamping groove (311) is provided in the other end of the first edge (31), and the other end of the second edge (32) is provided with a buckle (321), the other end of the first edge (31) and the other end of the second edge (32) are connected with the buckle (321) fastening the clamping groove (311) in a matched mode.

4. An absorbable and unidirectionally compressive intestinal stapler according to claim 2 or claim 3, wherein inner sides of the large ends are provided with a plurality of tooth-shaped structures (5) for preventing the intestinal canal from slipping off.

5. An absorbable and unidirectionally compressive intestinal stapler according to claim 4, wherein the elastic flared openings (4) are detachable.

## Patentansprüche

1. Eine absorbierbare und unidirektional komprimierende Darmklammer, umfassend einen ersten Rohrabschnitt (1) und einen zweiten Rohrabschnitt (2), die aufeinander abgestimmt sind und die aus einem für den Menschen anwendbaren abbaubaren Material hergestellt sind; wobei sich der erste Rohrabschnitt (1) relativ zu dem zweiten Rohrabschnitt (2) entlang einer gemeinsamen axialen Richtung vor und zurück bewegen kann, **dadurch gekennzeichnet, dass** der erste Rohrabschnitt (1) und der zweite Rohrabschnitt (2) untrennbar sind; der Rohrabschnitt (1) und der zweite Rohrabschnitt (2) jeweils ein großes Ende (11, 21) und ein kleines Ende umfassen, und die großen Enden (11, 21) zur Fixierung des Darmkanals dienen, und das kleine Ende des zweiten Rohrabschnitts (2) in das kleine Ende des ersten Rohrabschnitts (1) eingehüllt ist; die beiden großen Enden (11, 21) in ihrer Größe gleich sind; wenn sich der erste Rohrabschnitt (1) relativ zu dem zweiten Rohrabschnitt (2) bewegt, werden die beiden großen Enden (11, 21) nahe aneinander gezogen, um einen Darm/Darm- und eine Endstück/Endstück -Anastomose zu erreichen;
die Innenseite des großen Endes des ersten Rohrabschnitts (1) und die Innenseite des großen Endes des zweiten Rohrabschnitts (2) jeweils mit einem Ringvorsprung versehen sind, und eine Vielzahl durchgehender Nuten (22) auf der Wand-Innenseite des großen Endes des zweiten Rohrabschnitts (2) in Umfangsrichtung in Intervallen eingearbeitet sind, und eine Vielzahl in axialer Richtung angeordneter erster Zahnstangen (12) in Umfangsrichtung in Intervallen die Wand des kleinen Endes des ersten Rohrabschnitts (1) bilden, und die erste Zahnstange (12) die durchgehende Nut (22) durchdringt und entlang der durchgehenden Nut (22) bewegbar ist;
eine Vielzahl von zweiten Zahnstangen (23) in Intervallen in Umfangsrichtung die Wand des kleinen Endes des zweiten Rohrabschnitts (2) bilden, und an jedem Anschlag der ersten Zahnstangen (12) und der zweiten Zahnstangen (23) eine Gleitnut (13) vorhanden ist; die zweite Zahnstange (23) und die erste Zahnstange (12) versetzt angeordnet sind, die erste Zahnstange (12) und die zweite Zahnstange (23) aufeinander abgestimmt sind einen geschlossenen Raum zu bilden;
die hinteren Enden der ersten Zahnstangen (12) und der zweiten Zahnstangen (23) jeweils durch einen kreisförmigen Ring verbunden sind;
die Außenseite des kreisförmigen Rings an den Enden des kleinen Endes des ersten Rohrabschnitts (1) und denen des zweiten Rohrabschnitts (2) jeweils mit sich allmählich aufweitenden elastischen konischen Öffnungen (4) versehen sind; wenn der erste Rohrabschnitt (1) sich relativ zu dem zweiten Rohrabschnitt (2) schließt oder öffnet, öffnen sich die elastischen konischen Öffnungen (4) der beiden Enden oder werden in die großen Enden (11,) des ersten Rohrabschnitts (1) bzw. die großen Enden (21) des zweiten Rohrabschnitts (2) gequetscht.

2. Eine absorbierbare und unidirektional komprimierende Darmklammer nach Anspruch 1, weiterhin umfassend eine ringförmige Struktur (3), die in Umfangsrichtung angeordnet ist und zur Verstärkung der anastomotischen Stelle verwendet wird, die ringförmige Struktur (3) umfasst eine erste Flanke (31) und eine zweite Flanke (32), und ein Ende der ersten Flanke (31) ist an ein Ende der zweiten Flanke (32) klappbar angebracht, und das andere Ende der ersten Flanke (31) ist mit dem anderen Ende der zweiten Flanke (32) in geklemmter Weise verbunden.

3. Eine absorbierbare und unidirektional komprimierende Darmklammer nach Anspruch 2, wobei eine Klemmnut (311) in dem anderen Ende der ersten Flanke (31) vorgesehen ist und das andere Ende der zweiten Flanke (32) mit einem Verschluss (321) versehen ist, wobei das andere Ende der ersten Flanke (31) und das andere Ende der zweiten Flanke (32) mit dem Verschluss (321) verbunden werden, der in die Klemmnut (311) passend greift.

4. Eine absorbierbare und unidirektional komprimierende Darmklammer nach Anspruch 2 oder 3, wobei die Innenseite des großen Endes mit einer Vielzahl von zahnförmigen Strukturen (5) versehen ist, um ein Abrutschen des Darmkanals zu verhindern.

5. Eine absorbierbare und unidirektional komprimierende Darmklammer nach Anspruch 4, wobei die elastischen konischen Öffnungen (4) abnehmbar sind.

## Revendications

1. Agrafeuse pour anastomose d'intestin résorbable et compressible de manière unidirectionnelle, comprenant une première partie tuyau traversant (1) et une seconde partie tuyau traversant (2) qui sont accouplées l'une à l'autre et qui sont faites d'un matériau de dégradation acceptable par l'homme ; dans laquelle, la première partie tuyau traversant (1) peut se déplacer en va-et-vient par rapport à la seconde partie tuyau traversant (2) le long d'une direction axiale commune de celle-ci ; la première partie tuyau traversant (1) et la seconde partie tuyau traversant (2) sont inséparables ; la première partie tuyau traversant (1) et la seconde partie tuyau traversant (2) respectivement, comprennent de grandes extrémités (11, 21) et une petite extrémité, et les grandes extrémités (11, 21) permettent de fixer le canal intestinal, et la petite extrémité de la seconde partie tuyau traversant (2) est gainée dans la petite extrémité de la première partie tuyau traversant (1) ; les deux grandes extrémités (11, 21) sont de taille cohérente ; lorsque la première partie tuyau traversant (1) se déplace par rapport à la seconde partie tuyau traversant (2), les deux grandes extrémités (11, 21) sont attirées l'une à l'autre pour obtenir une anastomose intestin-intestin et de bout en bout ;
sur chacun d'un côté interne de la grande extrémité de la première partie tuyau traversant (1) et d'un côté interne de la grande extrémité de la seconde partie tuyau traversant (2) se trouve un cercle de saillies, et une pluralité de rainures traversantes (22) est disposée sur un côté interne d'une paroi de la grande extrémité de la seconde partie tuyau traversant (2) à intervalles dans la direction circonférentielle, une pluralité de premières crémaillères (12) à intervalles dans la direction circonférentielle forme une paroi de la petite extrémité de la première partie tuyau traversant (1) dans la direction axiale, la première crémaillère (12) pénètre à travers la rainure traversante (22) et est mobile le long de la rainure traversante (22) ;
une pluralité de secondes crémaillères (23) à intervalles dans la direction circonférentielle forme une paroi de la petite extrémité de la seconde partie tuyau traversant (2), et il existe une rainure coulissante (13) au niveau de chaque butée des premières crémaillères (12) et des secondes crémaillères (23) ; les secondes crémaillères (23) et les premières crémaillères (12) sont agencées en mode décalé, les premières crémaillères (12) et les secondes crémaillères (23) s'accouplent pour former un espace fermé ;
les extrémités de queue des premières crémaillères (12) et des secondes crémaillères (23) sont reliées par l'intermédiaire d'un anneau circulaire, respectivement ;
les côtés externes des anneaux circulaires au niveau des extrémités des petites extrémités de la première partie tuyau traversant (1) et de la seconde partie tuyau traversant (2) sont dotés d'ouvertures évasées élastiques pouvant se déployer progressivement (4), respectivement ;
lorsque la première partie tuyau traversant (1) se referme ou s'ouvre par rapport à la seconde partie tuyau traversant (2), les ouvertures évasées élastiques (4) des deux extrémités s'ouvrent ou sont écrasées dans la grande extrémité (11) de la première partie tuyau traversant (1) et la grande extrémité (21) de la seconde partie tuyau traversant (2) respectivement.

2. Agrafeuse pour anastomose d'intestin résorbable et compressible de manière unidirectionnelle selon la revendication 1, comprenant en outre une structure annulaire (3) qui est agencée dans le sens circonférentiel et est utilisée pour renforcer le site d'anastomose, la structure annulaire (3) comprend un premier bord (31) et un second bord (32), et une extrémité du premier bord (31) est articulée sur une extrémité du second bord (32), l'autre extrémité du premier bord (31) est reliée à l'autre extrémité du second bord (32) dans un mode serré.

3. Agrafeuse pour anastomose d'intestin résorbable et compressible de manière unidirectionnelle selon la revendication 2, dans laquelle une rainure de serrage (311) est prévue dans l'autre extrémité du premier bord (31), et l'autre extrémité du second bord (32) est dotée d'une boucle (321), l'autre extrémité du premier bord (31) et l'autre extrémité du second bord (32) sont reliées avec la boucle (321) fixant la rainure de serrage (311) dans un mode accouplé.

4. Agrafeuse pour anastomose d'intestin résorbable et compressible de manière unidirectionnelle selon la revendication 2 ou la revendication 3, dans laquelle des côtés internes des grandes extrémités sont dotés d'une pluralité de structures en forme de dents (5) pour empêcher que le canal intestinal glisse.

5. Agrafeuse pour anastomose d'intestin résorbable et compressible de manière unidirectionnelle selon la revendication 4, dans laquelle les ouvertures évasées élastiques (4) sont détachables.
